# EUROPÄISCHE PATENTSCHRIFT

(11) **EP 4 667 909 B1**
(45) Veröffentlichungstag und Bekanntmachung des Hinweises auf die Patenterteilung: **30.09.2026**
(21) Anmeldenummer: 24182615.5
(22) Anmeldetag: 17.06.2024
(51) Int. Cl.: G01N 21/3563, G01N 21/359, G01N 33/46, G01N 21/84

(54) **MESSVERFAHREN ZUR BESTIMMUNG DER MENGE AN ORGANISCHEN BESTANDTEILEN IN EINER HOLZASCHE**
METHOD FOR MEASURING THE AMOUNT OF ORGANIC CONSTITUENTS IN A WOOD ASH
PROCÉDÉ DE MESURE POUR DÉTERMINER LA QUANTITÉ DE COMPOSANTS ORGANIQUES DANS UNE CENDRE DE BOIS

(43) Veröffentlichungstag der Anmeldung: 24.12.2025
(73) Patentinhaber: Flooring Technologies Ltd., Kalkara SCM1001 (MT)
(72) Erfinder: Kalwa, Norbert, 32805 Horn-Bad Meinberg (DE); Zhang, Jinming, 10247 Berlin (DE); Seidack, Georg, 16909 Wittstock/Dosse (DE)
(74) Vertreter: Maikowski & Ninnemann Patentanwälte Partnerschaft mbB

(56) Entgegenhaltungen:
- CN-A- 112 432 910
- LABBE N ET AL: "Enhanced discrimination and calibration of biomass NIR spectral data using non-linear kernel methods", BIORESOURCE TECHNOLOGY, ELSEVIER, AMSTERDAM, NL, vol. 99, no. 17, 1 November 2008 (2008-11-01), pages 8445 - 8452, XP023182767, ISSN: 0960-8524, [retrieved on 20080414], DOI: 10.1016/J.BIORTECH.2008.02.052
- FELIX ENDRISS ET AL: "Analytical Methods for the Rapid Determination of Solid Biofuel Quality", CHEMIE INGENIEUR TECHNIK, WILEY VCH. VERLAG, WEINHEIM; DE, vol. 95, no. 10, 31 May 2023 (2023-05-31), pages 1503 - 1525, XP072501196, ISSN: 0009-286X, DOI: 10.1002/CITE.202200214

## Beschreibung

Die vorliegende Erfindung betrifft ein Verfahren zur Steuerung der Betriebsparameter einer Feuerungsanlage und eine Feuerungsanlage unter Verwendung von Daten ermittelt in einem Messverfahren zur Bestimmung der Menge an (unverbrannten) organischen Bestandteilen in einer Holzasche..

Biomasse, insbesondere Holz, wird seit alters her zur Gewinnung von Wärme und Energie durch Verbrennung verwendet. Im Verbrennungsprozess von Holz werden die meisten im Holz enthaltenen Stoffe oxidiert. Dabei entstehen Wärmeenergie als Hauptprodukt und Oxide als Nebenprodukt. Die Oxide des Kohlenstoffs und Stickstoffs entweichen als Gase (CO₂, NOₓ) in die Atmosphäre, während andere unverbrannte Inhaltsstoffe als Asche zurückbleiben. Die Asche, die im Verbrennungsofen liegen bleibt, wird als Rost- oder Grobasche bezeichnet, und stellt den mengenmäßig größten Anteil dar. Die anfallende Holzasche weist einen hohen pH-Wert auf (d.h. ist basisch) und muss als Abfallprodukt entsorgt werden.

Der Aschegehalt des Holzes schwankt in Abhängigkeit von der Holzart, den Wuchsbedingungen, der Fällungszeit u. a. und beträgt im Durchschnitt 0,3 bis 0,6 %; Rinden, Feinreisig, Nadeln und Blätter haben einen höheren Aschegehalt als das Holz.

Holzasche umfasst die mineralischen Bestandteile des Holzes, die bei der Verbrennung von Holz als unverbrennbare anorganische Bestandteile zurückbleiben als auch abhängig von den Verbrennungsbedingungen unverbrannte organische Bestandteile, insbesondere Lignin, Cellulose und Hemicelluose.

Vorherrschende anorganische Bestandteile in der Holzasche sind Alkali- und Erdalkalicarbonate (14 bis 19 % K₂O, Na₂O; 30 bis 40 %. CaO; 5 bis 11 %. MgO). Daneben kommen Eisen (1 bis 3 % Fe₂O₃), Phosphate (0,4 bis 5 % P₂O₅), Silicate (1,8 bis 3 % SiO₂), Sulfate (3 bis 5 % SO₃) vor.

Beim Betrieb von Kesselhäusern, die im Wesentlichen mit Biomasse (Holzabfälle, Pellets, Grünschnitt, Jährlingspflanzen, Klärschlämme usw.) betrieben werden, liegt ein Focus auf dem guten Ausbrand der organischen Bestandteile. Je nach Verbrennungstechnologie und eingesetztem Brennstoff können dabei Probleme auftreten. Ein guter Ausbrand ist erforderlich, damit die Asche deponiert werden kann. Sie muss aber, je nach Deponieklasse einen bestimmten Prozentsatz an unverbranntem Material unterschreiten. Dieser Prozentsatz liegt im Idealfall bei kleiner drei Prozent. Aschen, die höhere Prozentsätze an unverbranntem Material enthalten, dürfen auf bestimmten Deponien nicht gelagert werden oder vor einer Deponierung ist eine thermische Nachbehandlung erforderlich. Alle diese Maßnahmen erhöhen die Kosten für die Deponierung.

Durch die Aktivitäten auf europäischer Ebene möglichst keine zu entsorgenden Reststoffe anfallen zu lassen, sondern diese wieder einer Nutzung zu zuführen, werden an Aschen aus Feuerungen zunehmend höhere Qualitätsanforderungen gestellt. Diese müssen durch eine engmaschige Kontrolle und Überwachung sichergestellt werden. Gerade bei Aschen ist der bereits angesprochene Parameter der unverbrannten Bestandteile besonders wichtig, da z. B. bei Verwendung als Material im Straßenbau unverbranntes Material im Kontakt mit Grundwasser zu einer Kontamination führen kann.

Der Gehalt an organischen Bestandteilen/unverbrannten Bestandteilen wird durch die Bestimmung des Glühverlustes ermittelt. Dabei wird die Ascheprobe über einen definierten Zeitraum bei Temperaturen > 800°C erhitzt und dann der Masseverlust bestimmt. Dieses Verfahren ist aufwändig beginnend bei der Probenahme über die Einwaage, die Veraschung und die Auswaage vergehen u. U. Stunden. Dies ist gerade bei Feuerungen, die mit stückigen Holzabfällen betrieben werden, kritisch, da der Ausbrand durch verschiedene Parameter negativ beeinflusst werden kann. Zudem können lediglich punktuell Werte ermittelt werden, die nichts über die Gesamtsituation aussagen. Außerdem muss für diese Überwachung Personal und technische Ausstattung beschafft werden.

Daraus ergeben sich verschiedene Nachteile, wie fehlende kontinuierliche Überwachung, ein aufwändiges Verfahren verbunden mit hohen Kosten und zeitlichem Verzug.

Der Erfindung liegt daher die technische Aufgabe zu Grunde ein einfaches und schnelles Verfahren zur Verfügung zu stellen, um die oben geschilderten Probleme zu lösen. Es soll, wenn möglich eine kontinuierliche Überwachung der Aschen ermöglichen. Auch soll eine sofortige Übertragung der Werte sichergestellt werden, sodass bei sich ändernden Werten eine sofortige Reaktion möglich ist. Durch das Verfahren sollen zeitaufwändige und teure interne oder externe Analysen über Veraschungen überflüssig werden.

Diese Aufgabe wird erfindungsgemäß durch ein Verfahren mit den Merkmalen des Anspruchs 1 gelöst.

Entsprechend wird ein Verfahren zur Steuerung von Betriebsparametern einer Feuerungsanlage, in welcher Holzsache durch Verbrennen von Biomasse gewonnen wird umfassend ein Messverfahren zur Bestimmung der Menge an (unverbrannten) organischen Bestandteilen in einer Holzasche bereitgestellt, wobei das Messverfahren die folgenden Schritte umfasst:
- Bereitstellen von Ascheproben mit jeweils unterschiedlichen (bekannten, konventionell mittels Glühverlust bestimmten) quantitativen definierten Mengen an organischem Material als Referenzproben;
- Aufnahme von mindestens einem NIR-Spektrum von jeder der Referenzproben unter Verwendung von mindestens einem NIR-Messkopf in einem Wellenlängenbereich zwischen 1200 nm und 2000 nm, bevorzugt zwischen 1300 nm und 1800 nm, insbesondere bevorzugt zwischen 1400 nm und 1650 nm,
- Zuordnen der unterschiedlichen quantitativ definierten Mengen an organischem Material in den Referenzproben zu den aufgenommenen NIR-Spektren der genannten Referenzproben; und
- Erstellen eines Kalibriermodells für den Zusammenhang zwischen den spektralen Daten der NIR-Spektren und den dazugehörigen quantitativ definierten Mengen an organischem Material in den Referenzproben mittels einer multivariaten Datenanalyse;
- Bereitstellen von mindestens einer zu vermessenden Ascheproben mit einer unbekannten Menge an organischem Material, wobei die zu vermessende Ascheprobe nach dem Ausgang aus der Feuerungsanlage auf ein kontinuierlich laufendes Transportband gestreut wird und auf dem Transportband vermessen wird,
- Aufnehmen von mindestens einem NIR-Spektrum der bereitgestellten Ascheprobe unter Verwendung des mindestens einen NIR-Messkopfes in einem Wellenlängenbereich zwischen 1200 nm und 2000 nm, bevorzugt zwischen 1300 nm und 1800 nm, insbesondere bevorzugt zwischen 1400 nm und 1650 nm, und
- Bestimmen der quantitativen Menge des organischen Materials in der zu vermessenden Ascheprobe durch Vergleich des für die zu vermessende Ascheprobe aufgenommenen NIR-Spektrums mit dem aus den Referenzproben erstellten Kalibriermodell,
- wobei die Betriebsparameter der Feuerungsanlage ausgewählt sind aus Brennstoffzufuhr, Geschwindigkeit des Rostes, Luftzufuhr.

Das vorliegende Verfahren ermöglicht demnach die Bestimmung der unverbrannten, organischen Bestandteile in den Aschen mit Hilfe der NIR-Spektroskopie. Ein Beispiel für NIRspektroskopische Messungen zur Bestimmung des Ascheanteils und Verkohlungsanteils von verschiedenen Biomassen findet sich in dem Dokument von LABBE N ET AL: "Enhanced discrimination and calibration of biomass NIR spectral data using non-linear kernel methods",BIORESOURCE TECHNOLOGY, ELSEVIER, AMSTERDAM, NL, Bd. 99, Nr. 17, 1. November 2008 (2008-11-01), Seiten 8445-8452.

Gemäß der Erfindung erfolgt die Analyse bevorzugt kontinuierlich direkt hinter dem Ascheaustrag des Kesselhauses . Zu diesem Zweck wird ein NIR-Messkopf über der Transportvorrichtung für die Asche installiert. Wie später im Detail erläutert, kann der NIR-Messkopf über eine Rückkopplung die Parameter des Kesselhauses steuern (Brennstoffzufuhr, Geschwindigkeit Rost, Luftzufuhr usw.).

Des Weiteren ist vorteilhafterweise vorgesehen, dass der mindestens eine NIR-Messkopf mehrere Spektren pro Minute, bevorzugt bis zu acht Spektren pro Minute oder mehr aufnimmt, aus denen ein Mittelwert über die Anzahl der aufgenommenen Spektren gebildet wird.

Auch sollte der verwendete NIR-Messkopf unempfindlich gegenüber Temperaturschwankungen, Staub und Emissionen von Holzinhaltstoffen sein.

Das vorliegende Verfahren ermöglicht die Bereitstellung der Messwerte in kurzer Zeit (online, bevorzugt ohne störende Zeitverzögerung). Die Messdaten können zur Qualitätssicherung, Forschung und Entwicklung, zur Prozesskontrolle, Prozessregelung, Prozesssteuerung usw. eingesetzt werden. Durch den Messvorgang wird die Produktionsgeschwindigkeit usw. nicht reduziert. Grundsätzlich wird damit die Überwachung der Produktion verbessert. Zudem werden auch Stillstandszeiten durch Qualitätsbestimmungen und Anlagenjustierungen reduziert.

Das vorliegende Verfahren nutzt den Umstand aus, dass die NIR-Strahlung an der Oberfläche der Partikeloberfläche reflektiert bzw. gestreut wird. Die reflektierte bzw. gestreute NIR-Strahlung wird von dem NIR-Detektor erfasst, und das ermittelte NIR-Spektrum wird zur Bestimmung der gewünschten Parameter (hier Anteil des organischen Materials in Holzasche) verwendet.

In einer bevorzugten Ausführungsform des vorliegenden Messverfahrens ist vorgesehen, dass für die Bestimmung der Menge an in der Holzasche enthaltenen organischen Material spektrale Daten aus dem gesamten aufgenommenen Spektralbereich, insbesondere zwischen 1400 und 1650 nm, verwendet werden.

Gemäß dem erfindungsgemäßen Verfahren werden demnach zunächst Referenzproben von Holzasche bereitgestellt, die bekannte Mengen an organischem Material enthalten. Die Menge an unverbrannten organischen Material wird mittels konventioneller Methoden (Nachveraschung und Glühverlust) bestimmt.

Die Menge des organischen Materials in den Referenzproben beträgt zwischen 0 und 30 Gew%, bevorzugt zwischen 1 und 25 Gew%, besonders bevorzugt zwischen 2 und 20 Gew%, ganz besonders bevorzugt zwischen 3 und 17 Gew%, noch mehr bevorzugt zwischen 5 und 15 Gew% (bezogen auf die Gesamtmenge an Holzasche).

Von diesen Referenzproben werden zumindest ein NIR-Spektrum in einem Wellenlängenbereich zwischen 1200 nm und 2000 nm, bevorzugt zwischen 1300 nm und 1800 nm; insbesondere bevorzugt zwischen 1400 nm und 1650 nm aufgenommen.

Die unterschiedlichen quantitativen Mengen an organischem Material der Referenzproben werden dann den jeweils aufgenommen NIR-Spektren dieser Referenzproben zugeordnet und es wird ein Kalibriermodell für den Zusammenhang zwischen den spektralen Daten der NIR-Spektren der Referenzproben und den dazugehörigen Mengen an organischem Material in der Holzasche als Parameterwert mittels einer multivariaten Datenanalyse erstellt; d.h. zu jedem Parameterwert der Referenzprobe korrespondiert ein NIR-Spektrum der Referenzprobe. Die für die verschiedenen Parameter erstellten Kalibriermodelle werden in einem geeigneten Datenspeicher hinterlegt.

Anschließend wird mindestens eine zu vermessenden Ascheproben mit einer unbekannten Menge an organischem Material bereitgestellt, und mindestens ein NIR-Spektrum dieser Ascheprobe aufgenommen. Die quantitative Menge des in der Ascheprobe enthaltenen Menge an organischem Material kann durch Vergleich des für die Messprobe aufgenommenen NIR-Spektrums mit dem erstellten Kalibriermodell ermittelt werden.

Ein Vergleich und die Interpretation der NIR-Spektren erfolgt sinnvollerweise über den gesamten aufgenommenen Spektralbereich. Dies wird vorteilhaft mit einer an sich bekannten multivariaten Datenanalyse (MDA) durchgeführt. Bei multivariaten Analysemethoden werden in an sich bekannter Weise typischerweise mehrere statistische Variablen zugleich untersucht. Hierzu wird bei diesen Methoden üblicherweise die in einem Datensatz enthaltene Zahl von Variablen reduziert, ohne gleichzeitig die darin enthaltene Information zu mindern.

Im vorliegenden Fall erfolgt die multivariate Datenanalyse über das Verfahren der Partial Least Squares Regression (partielle Regression der kleinsten Quadrate, PLS) wodurch ein geeignetes Kalibrationsmodell erstellt werden kann. Die Auswertung der gewonnenen Daten wird bevorzugt mit einer geeigneten Analysesoftware vorgenommen wie z.B. mit der Analysesoftware SIMCA-P der Firma Umetrics AB oder The Unscrambler der Firma CAMO.

Die Bedeutung einer Wellenlänge für die Vorhersage von Parametern, wie der Menge an organischem Material, aus dem NIR-Spektrum wird mit Hilfe der Regressionskoeffizienten dargestellt. Dabei haben die Regionen mit großen Koeffizientenbeträgen starken Einfluss auf das Regressionsmodell. So zeigt die Darstellung der Regressionskoeffizienten in einem PLS-Regressionsmodell, dass der Wellenlängenbereich zwischen 1400 nm und 1650 nm für die Berechnung des Modells am wichtigsten ist, da hier die Beträge der Regressionskoeffizienten am größten sind. Die anderen Bereiche im Spektrum haben zwar geringeren Informationsgehalt in Bezug auf die NIR-Messung, tragen aber dennoch dazu bei, die weiteren Informationen bzw. störenden Einflussgrößen zu berücksichtigen bzw. zu minimieren.

Zur Eliminierung von störenden Einflüssen kann es notwendig sein, die spektralen Daten mit mathematischen Vorbehandlungsmethoden (z. B. derivative Datenvorbehandlung, Standardisierung gemäß SNVT (Standard Normal Variate Transformation), multiplikative Signal-Korrektur (EMSC, Extended Multiplicative Signal Correction usw.) zu bearbeiten.

Wie bereits oben angedeutet, umfasst das in der Holzasche enthaltene organische Material Lignin, Zellulose und/oder Hemizellulose.

Die Menge des in der Holzasche zu bestimmenden organischen Materials beträgt zwischen 0 und 30 Gew%, bevorzugt zwischen 1 und 25 Gew%, besonders bevorzugt zwischen 2 und 20 Gew%, ganz besonders bevorzugt zwischen 3 und 17 Gew%, noch mehr bevorzugt zwischen 5 und 15 Gew% (bezogen auf die Gesamtmenge an Holzasche).

Als anorganische Bestandteile weist die zu vermessende Holzasche insbesondere Calcium, Magnesium, Kalium, Eisen, Phosphate, Silicate, Sulfate auf. Typische anorganische Bestandteile sind Alkali- und Erdalkalicarbonate (14 bis 19 % K₂O, Na₂O; 30 bis 40 %. CaO; 5 bis 11 %. MgO), Eisen (1 bis 3 % Fe₂O₃), Phosphate (0,4 bis 5 % P₂O₅), Silicate (1,8 bis 3 % SiO₂), Sulfate (3 bis 5 % SO₃).

Das vorliegende Messverfahren zur Bestimmung der Menge an (unverbrannten) organischen Bestandteilen in einer Holzasche erfolgt bevorzugt kontinuierlich online oder offline nach Ausgang der Holzasche aus einer Feuerungsanlage.

Die zu analysierende Asche kann kontinuierlich als Teilstrom aus der Gesamtmenge der Asche ausgeschleust werden oder in regelmäßigen Abständen, z.B. 1-2 / h) als Einzelprobe, entnommen werden

Bevorzugt wird die Holzasche, insbesondere ein Teilstrom der ausgeschleusten Holzasche, nach dem Ausgang aus der Feuerungsanlage auf ein kontinuierlich laufendes Transportband gestreut und auf dem Transportband vermessen.

Vor der NIR-Messung wird die Holzasche nach Austritt aus der Feuerungsanlage gemahlen. Nach dem Mahlprozess weist die die zu vermessende Holzasche eine mittlere Partikelgröße von 10 bis 1000 µm, bevorzugt 30 bis 700 µm, insbesondere bevorzugt 50 bis 500 µm, noch bevorzugter 100 bis 300 µm auf.

Die Schichtdicke der auf das Transportband aufgestreuten Asche wird durch das installierte Band vorgegeben. Typische Schichtdicken an aufgestreuter Holzsache liegen zwischen 1 mm und 100 mm, bevorzugt zwischen 10 und 80 mm, insbesondere bevorzugt 30 und 50 µm.

In einer weitergehenden Ausführungsform des vorliegenden Verfahrens ist vorgesehen, dass der mindestens eine NIR-Messkopf sich quer zur Laufrichtung des mit der Holzasche bestreuten Transportbandes bewegt und über die gesamte Breite des Transportbandes traversiert.

Es wird somit ein Verfahren bereitgestellt, in welchem durch die Verwendung eines NIR-Messkopfes, die Menge an unverbrannten, organischem Material in Holzsache aus einem einzigen NIR-Spektrum bzw. der Reflektion bzw. Streuung von NIR-Strahlung bestimmt werden kann, und zwar durch eine berührungslose Messung. Die mit dem Messkopf oder den Messköpfen ermittelten Daten werden in einer vorteilhaften Ausprägung der Erfindung direkt zur Anlagensteuerung oder -regelung verwendet.

Erfindungsgemäß werden die mit dem vorliegenden Messverfahren bestimmten Daten zur Steuerung der Betriebsparameter einer Feuerungsanlage (z.B. ein Kesselhaus) verwendet, in welcher die Holzasche durch Verbrennen von Biomasse, insbesondere Holz, gewonnen wird.

Die Parameter zur Steuerung der Betriebsparameter einer Feuerungsanlage sind dabei ausgewählt aus Brennstoffzufuhr, Geschwindigkeit des Rostes, Luftzufuhr.

Die Vorteile des vorliegenden Verfahrens sind vielfältig: Berührungslose Multiparameterbestimmung ("real time"- oder "Echtzeit"-Messung) mit deutlich reduzierter zeitlicher Verzögerung in der Auswertung der gemessenen Parameterwerte; verbesserte Anlagensteuerung bzw. -regelung, Verringerung des Ausschusses, Verbesserung der Qualität der auf der Anlage hergestellten Produkte, Kostenreduzierung und Verbesserung der Anlagenverfügbarkeit.

Erfindungsgemäß wird daher ebenfalls eine Feuerungsanlage zur Verbrennung von Biomasse, insbesondere von Holz, bereitgestellt, wobei die Feuerungsanlage mit folgenden Elementen verbunden ist:
- Mindestens einem NIR-Multimesskopf zur Aufnahme von mindestens einem NIR-Spektrum einer die Feuerungsanlage verlassenden Holzasche in einem Wellenlängenbereich zwischen 1200 nm und 2000 nm, bevorzugt zwischen 1300 nm und 1800 nm, insbesondere bevorzugt zwischen 1400 nm und 1650 nm,
- mindestens ein Steuerungssystem zur Steuerung der Feuerungsanlage, wobei das Steuerungssystem der Feuerungsanlage mindestens eine computergestützte Auswerteeinheit und eine Datenbank umfasst,
- wobei der mindestens eine NIR-Multimesskopf jeweils mit dem mindestens einem Steuerungssystem mit Auswerteeinheit und Datenbank zur Prozessierung und Speicherung der aufgenommenen NIR-Daten verbunden ist,
- wobei die Auswerteeinheit konfiguriert ist, die Menge an organischem Material in der zu vermessenden Holzascheprobe durch einen automatisierten Vergleich des für die Holzascheprobe aufgenommenen NIR-Spektrums mit einem erstellten Kalibriermodell zu bestimmen, wobei das Kalibriermodell anhand von Referenzproben gemäß Anspruch 1 bestimmt wird;
- wobei die Datenbank konfiguriert ist, die so bestimmten Daten zu speichern, und
- wobei die bestimmten Daten zur Steuerung von Betriebsparametern der Feuerungsanlage verwendet werden., wobei die Betriebsparameter der Feuerungsanlage ausgewählt sind aus Brennstoffzufuhr, Geschwindigkeit des Rostes, Luftzufuhr.

Der NIR-Multimesskopf ist dabei so konfiguriert, dass die gemessenen Parameter (Ist-Werte) an die Auswerte-Einheit geliefert werden, die bei Abweichung der gemessenen Parameter (Ist-Werte) von den entsprechenden Soll-Werten dieser Parameter den Produktionsablauf entsprechend regelt oder vorausschauend steuert.

Zudem ist das Steuerungssystem konfiguriert, um bei Abweichungen der gemessenen Parameter (Ist-Werte) von den Soll-Werten dieser Parameter eine automatische Anpassung durch die Anlagensteuerung vorzunehmen.

Für den Abgleich und die Steuerung der jeweiligen Anlage wird ein computerimplementiertes Verfahren sowie ein Computerprogramm umfassend Befehle, die bei der Ausführung des Programms durch einen Computer diesen veranlassen das computerimplementierte Verfahren auszuführen, bereitgestellt. Das Computerprogramm ist in einer Speichereinheit des Steuerungssystems der jeweiligen Fertigungslinie gespeichert.

Die Erfindung wird nachfolgend an Ausführungsbeispielen unter Bezugnahme auf die Figuren näher erläutert. Es zeigen:

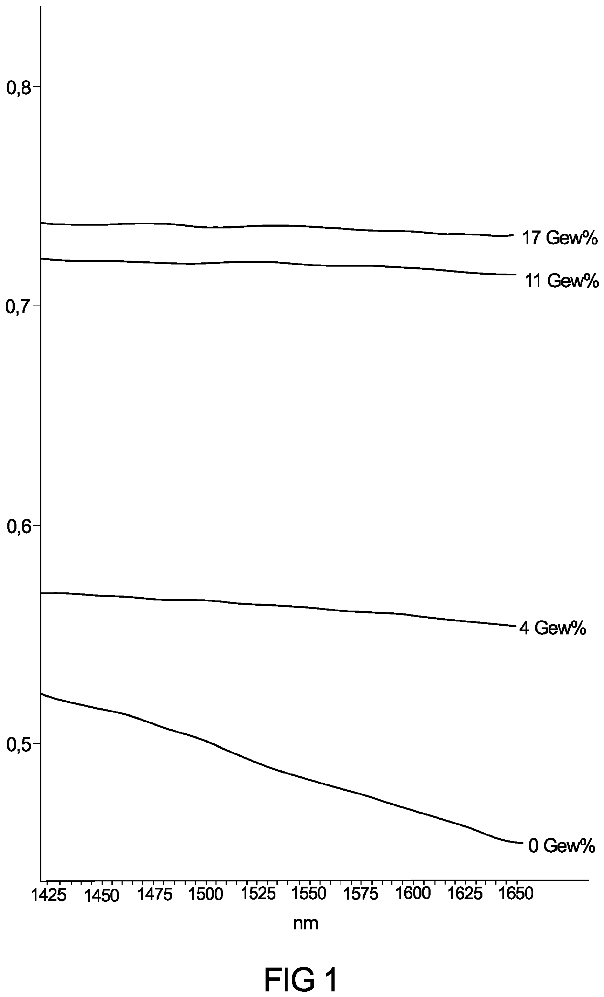

- Figur 1: NIR-Spektren aufgenommen von Probe von Holzasche mit unterschiedlichen Anteilen von organischem Material (0 Gew%, 4 Gew%, 11 Gew% und 17Gew%)

### Ausführungsbeispiel 1:

Verschiedene Kesselascheproben, deren Gehalt an unverbranntem organischen Material zunächst durch eine Nachverbrennung im Muffelofen ( 800°C, 4 h ) bestimmt wurde, wurden mit einem NIR-Messkopf analysiert. Dabei wurden aus jeder Ascheprobe drei Einzelproben erzeugt und diese vermessen. Die Nachveraschung zeigte Werte an unverbranntem, organischen Material von 0 Gew%, 4 Gew%, 11 Gew% und 17 Gew%.

Bei den NIR-Spektren (siehe Figur 1) waren abhängig vom Gehalt an organischen Material 0 Gew%, 4 Gew%, 11 Gew% und 17 Gew%) deutliche Unterschiede erkennbar. Aus diesen Spektren wurde über eine Kalibrationssoftware ein Ausgleichsgerade berechnet.

Anschließend wurden nachveraschte Proben mit Kesselascheproben, deren Gehalt an organischem Material bekannt war gemischt und erneut mit einem NIR-Messkopf vermessen.

Es zeigte sich, dass mit dem Messkopf der erwartete Messwert mit einem Fehler von kleiner 10% bestimmt wurde.

### Ausführungsbeispiel 2:

Nachveraschte Kesselasche wurde mit Schleifstaub aus dem Schliff von HDF-Platten homogen vermischt. Zu der Asche wurden 5, 10, 15 und 20 Gew% Schleifstaub zugegeben.

Dann wurde mit Hilfe des NIR-Messkopfes eine Bestimmung an unverbranntem, organischen Material durchgeführt. Es ergaben sich folgende Werte:

| Probe Kesselasche | Zugabe Schleifstaub in Gew% | Ermittelte Menge an organischen Material via NIR in Gew% |
|---|---|---|
| 1 | 5 | 4,6 |
| 2 | 10 | 9.5 |
| 3 | 15 | 14,1 |
| 4 | 20 | 19,0 |

Die Minderbefunde können aus dem Gehalt an Leim im Schleifstaub resultieren, welcher in der Kesselasche niedriger liegen sollte.

Es ergeben sich Vorteile:
- Kontinuierliche Überwachung
- Kostenreduzierungen
- Schnellere Analytik
- Steuerung Kesselhaus

## Patentansprüche

1. Verfahren zur Steuerung von Betriebsparametern einer Feuerungsanlage, in welcher Holzasche durch Verbrennen von Biomasse gewonnen wird, umfassend ein Messverfahren zur Bestimmung der Menge an organischen Bestandteilen in der Holzasche, wobei das Messverfahren die folgender Schritte umfasst:
- Bereitstellen von Ascheproben mit jeweils unterschiedlichen, quantitativ definierten Mengen an organischen Material als Referenzproben;
- Aufnahme von mindestens einem NIR-Spektrum von jeder der Referenzproben unter Verwendung von mindestens einem NIR-Messkopf in einem Wellenlängenbereich zwischen 1200 nm und 2000 nm, bevorzugt zwischen 1300 nm und 1800 nm, insbesondere bevorzugt zwischen 1400 nm und 1650 nm
- Zuordnen der unterschiedlichen quantitativ definierten Mengen an organischem Material in den Referenzproben zu den aufgenommenen NIR-Spektren der genannten Referenzproben; und
- Erstellen eines Kalibriermodells für den Zusammenhang zwischen den spektralen Daten der NIR-Spektren und den dazugehörigen quantitativ definierten Mengen an organischem Material in den Referenzproben mittels einer multivariaten Datenanalyse;
- Bereitstellen von mindestens einer zu vermessenden Ascheprobe mit einer unbekannten Menge an organischem Material, wobei die zu vermessende Ascheprobe nach dem Ausgang aus der Feuerungsanlage auf ein kontinuierlich laufendes Transportband gestreut wird und auf dem Transportband vermessen wird,
- Aufnehmen von mindestens einem NIR-Spektrum der bereitgestellten zu vermessenden Ascheprobe unter Verwendung des mindestens einen NIR-Messkopfes in einem Wellenlängenbereich zwischen 1200 nm und 2000 nm, bevorzugt zwischen 1300 nm und 1800 nm, insbesondere bevorzugt zwischen 1400 nm und 1650 nm, und
- Bestimmen der quantitativen Menge des organischen Materials in der zu vermessenden Ascheprobe durch Vergleich des für die zu vermessende Ascheprobe aufgenommenen NIR-Spektrums mit dem aus den Referenzproben erstellten Kalibriermodell,
- wobei die Betriebsparameter der Feuerungsanlage ausgewählt sind aus Brennstoffzufuhr, Geschwindigkeit des Rostes, Luftzufuhr.

2. Verfahren nach Anspruch 1, **dadurch gekennzeichnet, dass** für die Bestimmung der Menge an in der Holzasche enthaltenen organischen Material spektrale Daten aus dem gesamten aufgenommenen Spektralbereich, insbesondere zwischen 1400 und 1650 nm, verwendet werden.

3. Verfahren nach einem der vorhergehenden Ansprüche, **dadurch gekennzeichnet, dass** die Menge des in der Holzasche zu bestimmenden organischen Materials zwischen 0 und 30 Gew%, bevorzugt zwischen 1 und 25 Gew%, besonders bevorzugt zwischen 2 und 20 Gew%, ganz besonders bevorzugt zwischen 3 und 17 Gew%, noch mehr bevorzugt zwischen 5 und 15 Gew% (bezogen auf die Gesamtmenge an Holzasche) beträgt.

4. Verfahren nach einem der vorhergehenden Ansprüche, **dadurch gekennzeichnet, dass** das in der Holzasche enthaltene organische Material Lignin, Zellulose und/oder Hemizellulose umfasst,

5. Verfahren nach einem der vorhergehenden Ansprüche, **dadurch gekennzeichnet, dass** die zu vermessende Holzasche anorganische Bestandteile, insbesondere Calcium, Magnesium, Kalium, Eisen, Phosphate, Silicate, Sulfate aufweist.

6. Verfahren nach einem der vorhergehenden Ansprüche, **dadurch gekennzeichnet, dass** die Bestimmung der Menge an in der Holzasche enthaltenen organischem Material in Holzasche kontinuierlich online oder offline erfolgt nach Ausgang der Holzasche aus einer Feuerungsanlage erfolgt.

7. Verfahren nach einem der vorhergehenden Ansprüche, **dadurch gekennzeichnet, dass** die Holzasche nach Austritt aus der Feuerungsanlage gemahlen wird.

8. Verfahren nach einen der vorhergehenden Ansprüche, **dadurch gekennzeichnet, dass** die zu vermessende Holzasche eine mittlere Partikelgröße von 10 bis 1000 µm, bevorzugt 30 bis 700 µm, insbesondere bevorzugt 50 bis 500 µm, noch bevorzugter 100 bis 300 µm aufweist.

9. Verfahren nach einem der vorhergehenden Ansprüche **dadurch gekennzeichnet, dass** die Holzasche durch Verbrennen von Holz, gewonnen wird.

10. Feuerungsanlage zur Verbrennung von Biomasse, insbesondere von Holz, verbunden mit
- Mindestens einem NIR-Multimesskopf zur Aufnahme von mindestens einem NIR-Spektrum einer die Feuerungsanlage verlassenden Holzsasche in einem Wellenlängenbereich zwischen 1200 nm und 2000 nm, bevorzugt zwischen 1300 nm und 1800 nm, insbesondere bevorzugt zwischen 1400 nm und 1650 nm,
- mindestens ein Steuerungssystem zur Steuerung der Feuerungsanlage, wobei das Steuerungssystem der Feuerungsanlage mindestens eine computergestützte Auswerteeinheit und eine Datenbank umfasst,
- wobei der mindestens eine NIR-Multimesskopf jeweils mit dem mindestens einem Steuerungssystem mit Auswerteeinheit und Datenbank zur Prozessierung und Speicherung der aufgenommenen NIR-Daten verbunden ist,
- wobei die Auswerteeinheit konfiguriert ist, die Menge an organischem Material in der zu vermessenden Holzascheprobe durch einen automatisierten Vergleich des für die Holzascheprobe aufgenommenen NIR-Spektrums mit einem erstellten Kalibriermodell zu bestimmen, wobei das Kalibriermodell anhand von Referenzproben gemäß Anspruch 1 bestimmt wird;
- wobei die Datenbank konfiguriert ist, die so bestimmten Daten zu speichern, und
- wobei die bestimmten Daten zur Steuerung von Betriebsparametern der Feuerungsanlage verwendet werden, wobei die Betriebsparameter der Feuerungsanlage ausgewählt sind aus Brennstoffzufuhr, Geschwindigkeit des Rostes, Luftzufuhr.

11. Feuerungsanlage nach Anspruch 10, **dadurch gekennzeichnet, dass** der NIR-Multimesskopf konfiguriert ist, die gemessenen Parameter (Ist-Werte) an die Auswerte-Einheit zu liefern, die bei Abweichung der gemessenen Parameter (Ist-Werte) von den entsprechenden Soll-Werten dieser Parameter den Produktionsablauf entsprechend regelt oder vorausschauend steuert.

12. Feuerungsanlage nach Anspruch 10 oder 11, **dadurch gekennzeichnet, dass** das Steuerungssystem konfiguriert ist, bei Abweichungen der gemessenen Parameter (Ist-Werte) von den Soll-Werten dieser Parameter eine automatische Anpassung durch die Anlagensteuerung vorzunehmen.

## Claims

1. Method for controlling operating parameters of a combustion plant in which wood ash is obtained by burning biomass, comprising a measuring method for determining the amount of organic constituents in a wood ash, wherein the measuring method comprises the following steps:
- providing ash samples each with different, quantitatively defined amounts of organic material as reference samples;
- recording at least one NIR spectrum of each of the reference samples using at least one NIR measuring head in a wavelength range between 1200 nm and 2000 nm, preferably between 1300 nm and 1800 nm, in particular preferably between 1400 nm and 1650 nm
- assigning the different quantitatively defined amounts of organic material in the reference samples to the recorded NIR spectra of said reference samples; and
- creating a calibration model for the relationship between the spectral data of the NIR spectra and the corresponding quantitatively defined amounts of organic material in the reference samples by means of multivariate data analysis;
- providing at least one ash sample to be measured with an unknown amount of organic material, wherein the ash sample to be measured is spread on a continuously running conveyor belt after leaving the furnace and is measured on the conveyor belt,
- recording at least one NIR spectrum of the provided ash sample to be measured using the at least one NIR measuring head in a wavelength range between 1200 nm and 2000 nm, preferably between 1300 nm and 1800 nm, in particular preferably between 1400 nm and 1650 nm, and
- determining the quantitative amount of organic material in the ash sample to be measured by comparing the NIR spectrum recorded for the ash sample to be measured with the calibration model created from the reference samples,
- wherein the operating parameters of the combustion plant are selected from fuel supply, speed of the grate, air supply.

2. Method according to claim 1, **characterized in that** spectral data from the entire recorded spectral range, in particular between 1400 and 1650 nm, are used to determine the amount of organic material contained in the wood ash.

3. Method according to one of the preceding claims, **characterized in that** the amount of organic material to be determined in the wood ash is between 0 and 30 wt%, preferably between 1 and 25 wt%, particularly preferably between 2 and 20 wt%, very particularly preferably between 3 and 17 wt%, even more preferably between 5 and 15 wt% (based on the total amount of wood ash).

4. Method according to one of the preceding claims, **characterized in that** the organic material contained in the wood ash comprises lignin, cellulose and/or hemicellulose,

5. Method according to one of the preceding claims, **characterized in that** the wood ash to be measured comprises inorganic constituents, in particular calcium, magnesium, potassium, iron, phosphates, silicates, sulphates.

6. Method according to one of the preceding claims, **characterized in that** the determination of the amount of organic material contained in the wood ash is carried out continuously online or offline after the wood ash has been discharged from a combustion plant.

7. Method according to one of the preceding claims, **characterized in that** the wood ash is ground after leaving the combustion plant.

8. Method according to one of the preceding claims, **characterized in that** the wood ash to be measured has an average particle size of 10 to 1000 µm, preferably 30 to 700 µm, more preferably 50 to 500 µm, even more preferably 100 to 300 µm.

9. Method according to one of the preceding claims, **characterized in that** the wood ash is obtained by burning wood.

10. Combustion plant for the combustion of biomass, in particular wood, combined with
- at least one NIR multi-measuring head for recording at least one NIR spectrum of a wood ash leaving the combustion plant in a wavelength range between 1200 nm and 2000 nm, preferably between 1300 nm and 1800 nm, in particular preferably between 1400 nm and 1650 nm,
- at least one control system for controlling the combustion plant, wherein the control system of the combustion plant comprises at least one computer-aided evaluation unit and a database,
- wherein the at least one NIR multi-measuring head is connected to the at least one control system with evaluation unit and database for processing and storing the recorded NIR data,
- wherein the evaluation unit is configured to determine the amount of organic material in the wood ash sample to be measured by an automated comparison of the NIR spectrum recorded for the wood ash sample with a created calibration model, wherein the calibration model is determined on the basis of reference samples according to claim 1;
- wherein the database is configured to store the data so determined, and
- wherein the determined data are used to control operating parameters of the combustion plant, wherein the operating parameters are selected from fuel supply, speed of the grate, air supply.

11. Combustion plant according to claim 10, **characterized in that** the NIR multi-measuring head is configured to supply the measured parameters (actual values) to the evaluation unit, which controls or anticipates the production process accordingly if the measured parameters (actual values) deviate from the corresponding target values of these parameters.

12. Combustion plant according to claim 10 or 11, **characterized in that** the control system is configured to make an automatic adjustment by the plant control system in the event of deviations of the measured parameters (actual values) from the setpoint values of these parameters.

## Revendications

1. Procédé de commande de paramètres de fonctionnement d'une installation de combustion dans laquelle des cendres de bois sont obtenues par combustion de biomasse, comprenant un procédé de mesure pour déterminer la quantité de composés organiques dans les cendres de bois, dans lequel le procédé de mesure comprend les étapes suivantes :
- la mise à disposition d'échantillons de cendres avec des quantités différentes et quantitativement définies de matières organiques comme échantillons de référence ;
- l'enregistrement d'au moins un spectre NIR de chacun des échantillons de référence en utilisant au moins une tête de mesure NIR dans une plage de longueurs d'onde entre 1200 nm et 2000 nm, et de manière préférée entre 1300 nm et 1800 nm, en particulier de manière préférée entre 1400 nm et 1650 nm ;
- l'association des quantités définies quantitativement différemment de matières organiques dans les échantillons de référence aux spectres NIR enregistrés desdits échantillons de référence ; et
- la création d'un modèle d'étalonnage pour la corrélation entre les données spectrales des spectres NIR et les quantités définies quantitativement associées de matières organiques dans les échantillons de référence au moyen d'une analyse de données multivariée ;
- la mise à disposition d'au moins un échantillon de cendres à mesurer avec une quantité inconnue de matières organiques, dans lequel l'échantillon de cendres à mesurer est dispersé après la sortie de l'installation de combustion sur une bande transporteuse à fonctionnement continu et est mesuré sur la bande transporteuse ;
- l'enregistrement d'au moins un spectre NIR de l'échantillon de cendres à mesurer mis à disposition en utilisant l'au moins une tête de mesure NIR dans une plage de longueurs d'onde entre 1200 nm et 2000 nm, de manière préférée entre 1300 nm et 1800 nm, en particulier de manière préférée entre 1400 nm et 1650 nm ; et
- la détermination de la quantité quantitative de matières organiques dans l'échantillon de cendres à mesurer en comparant le spectre NIR enregistré pour l'échantillon de cendres à mesurer au modèle d'étalonnage créé à partir des échantillons de référence,
- dans lequel les paramètres de fonctionnement de l'installation de combustion sont choisis parmi l'alimentation en combustible, la vitesse de la grille, l'alimentation en air.

2. Procédé selon la revendication 1, **caractérisé en ce que** des données spectrales de l'ensemble de la plage spectrale enregistrée, en particulier entre 1400 et 1650 nm, sont utilisées pour déterminer la quantité de matières organiques contenues dans les cendres de bois.

3. Procédé selon l'une quelconque des revendications précédentes, **caractérisé en ce que** la quantité de matières organiques à déterminer dans les cendres de bois est comprise entre 0 et 30 % en poids, de manière préférée entre 1 et 25 % en poids, de manière particulièrement préférée entre 2 et 20 % en poids, de manière très particulièrement préférée entre 3 et 17 % en poids, de manière encore plus préférée entre 5 et 15 % en poids (par rapport à la quantité totale de cendres de bois).

4. Procédé selon l'une quelconque des revendications précédentes, **caractérisé en ce que** les matières organiques contenues dans les cendres de bois comprennent de la lignine, de la cellulose et/ou de l'hémicellulose.

5. Procédé selon l'une quelconque des revendications précédentes, **caractérisé en ce que** les cendres de bois à mesurer contiennent des composants inorganiques, en particulier du calcium, du magnésium, du potassium, du fer, du phosphate, des silicates, des sulfates.

6. Procédé selon l'une quelconque des revendications précédentes, **caractérisé en ce que** la détermination de la quantité de matières organiques contenues dans les cendres de bois dans des cendres de bois est effectuée en continu en ligne ou hors ligne après la sortie des cendres de bois d'une installation de combustion.

7. Procédé selon l'une quelconque des revendications précédentes, **caractérisé en ce que** les cendres de bois sont broyées après la sortie de l'installation de combustion.

8. Procédé selon l'une quelconque des revendications précédentes, **caractérisé en ce que** les cendres de bois à mesurer présentent une taille moyenne de particules de 10 à 1000 µm, de manière préférée de 30 à 700 µm, en particulier de manière préférée de 50 à 500 µm, de manière encore plus préférée de 100 à 300 µm.

9. Procédé selon l'une quelconque des revendications précédentes, **caractérisé en ce que** les cendres de bois sont obtenues par combustion de bois.

10. Installation de combustion pour la combustion de biomasse, en particulier de bois, associée à
- au moins une tête de multimesure NIR pour l'enregistrement d'au moins un spectre NIR de cendres de bois sortant de l'installation de combustion dans une plage de longueurs d'onde entre 1200 nm et 2000 nm, de manière préférée entre 1300 nm et 1800 nm, en particulier de manière préférée entre 1400 nm et 1650 nm,
- au moins un système de commande pour la commande de l'installation de combustion, dans laquelle le système de commande de l'installation de combustion comprend au moins une unité d'évaluation assistée par ordinateur et une base de données,
- dans laquelle l'au moins une tête de multimesure NIR est reliée respectivement à l'au moins un système de commande avec une unité d'évaluation et une base de données pour le traitement et le stockage des données NIR enregistrées,
- dans laquelle l'unité d'évaluation est configurée pour déterminer la quantité de matières organiques dans l'échantillon de cendres de bois à mesurer par comparaison automatisée du spectre NIR enregistré pour l'échantillon de cendres de bois à un modèle d'étalonnage créé, dans laquelle le modèle d'étalonnage est déterminé à l'aide d'échantillons de référence selon la revendication 1 ;
- dans laquelle la base de données est configurée pour stocker les données ainsi déterminées,
- dans laquelle les données déterminées sont utilisées pour commander des paramètres de fonctionnement de l'installation de combustion, dans laquelle les paramètres de fonctionnement de l'installation de combustion sont choisis parmi l'alimentation en combustible, la vitesse de la grille, l'alimentation en air.

11. Installation de combustion selon la revendication 10, **caractérisée en ce que** la tête de multimesure NIR est configurée pour fournir les paramètres mesurés (valeurs réelles) à l'unité d'évaluation, qui régule de manière correspondante ou commande de manière anticipée le déroulement de la production en cas d'écart des paramètres mesurés (valeurs réelles) par rapport aux valeurs de consigne correspondantes desdits paramètres.

12. Installation de combustion selon la revendication 10 ou 11, **caractérisée en ce que** le système de commande est configuré pour réaliser une adaptation automatique par la commande d'installation en cas d'écarts des paramètres mesurés (valeurs réelles) par rapport aux valeurs de consigne desdits paramètres.
